# DEMANDE DE BREVET EUROPEEN

(11) **EP 3 733 294 A1**
(43) Date de publication de la demande: **04.11.2020**
(21) Numéro de dépôt: 20170983.9
(22) Date de dépôt: 23.04.2020
(51) Int. Cl.: B01L 9/00

(54) **DISPOSITIF POUR LE STOCKAGE ET LA TRAÇABILITÉ DE SUPPORTS D'ÉCHANTILLONS BIOLOGIQUES**

(30) Priorité: 02.05.2019 FR 1904636
(71) Demandeur: Dreampath Diagnostics, 67100 Strasbourg (FR)
(72) Inventeur: JORDAN NAVAS, Pablo, 28005 Madrid (ES); CREPIEUX, Thomas, 67000 Strasbourg (FR); WILHELM, Valérie, 67114 Eschau (FR); HIRTZ, Thomas, 67330 Bosselshausen (FR)
(74) Mandataire: Hugues, Catherine

(57) **Abrégé**

La présente invention concerne un dispositif pour le stockage et la traçabilité d'échantillons biologiques (1) placés dans des supports (3) munis d'informations d'identification sous forme de données encodées (2), ledit dispositif comportant :
- au moins un plateau (4) présentant une pluralité d'alvéoles (5), formant des logements de stockage desdits supports (3),
- au moins un appareil de lecture des données encodées et de détermination de données relatives à la localisation de chaque support (3) au sein du plateau (4),
- des moyens de traitement informatiques des données lues et déterminées par ledit appareil.

Il est caractérisé en ce qu'il comporte des moyens de reconnaissance d'un logement (5) vide au sein dudit plateau (4) définis par des moyens de détection d'une caractéristique physique du fond (9) de l'alvéole (5) ou d'un élément solidaire de ce fond (9) .

## Description

La présente invention a pour objet un dispositif pour le stockage et la traçabilité d'échantillons biologiques placés dans des supports munis d'informations d'identification sous forme de données encodées, ledit dispositif comportant :
- au moins un réceptacle pourvu de logements de stockage desdits supports, le réceptacle étant défini par un plateau présentant une pluralité d'alvéoles formant lesdits logements, configurées pour héberger chacune un support d'échantillon biologique, délimitées par un fond surmonté par une paroi périphérique,
- au moins un appareil de lecture des données encodées et de détermination de données relatives à la localisation de chaque support au sein du réceptacle, comportant au moins une cavité conformée pour accueillir le réceptacle de manière temporaire, ainsi que
- des moyens de traitement informatiques des données lues et déterminées par ledit appareil.

Dans le cadre de la prise en charge médicale d'un patient ou celui de travaux de recherche dans le domaine humain, animal ou végétal, des prélèvements tissulaires ou cellulaires sont couramment effectués en vue d'une analyse histologique et/ou moléculaire, destinée ou non à l'établissement d'un diagnostic.

Afin de permettre une parfaite visualisation au microscope des structures cellulaires ou tissulaires analysées, de tels prélèvements font l'objet d'une préparation préalable comportant différentes étapes. De manière résumée, au cours de ces dernières, les échantillons frais prélevés sont placés dans des cassettes ajourées, subissent une phase de déshydratation, puis une inclusion en paraffine conduisant à des blocs d'échantillons biologiques. Des coupes fines et régulières, prélevées à partir de ces derniers, sont ensuite placées sur des lames, subissent encore différents traitements destinés à colorer les éléments structurels à observer, et sont enfin recouvertes d'une résine puis d'une lamelle permettant leur lecture au microscope et assurant leur protection.

Toutes ces étapes de préparation des échantillons biologiques en vue de leur analyse supposent de nombreuses manipulations au cours desquelles des mesures pour en assurer la traçabilité, doivent être mises en œuvre.

Il en va de même lors des phases d'analyse, pendant lesquelles les lames échantillonnées sont amenées à circuler d'un poste vers un autre au sein d'un même laboratoire, ou entre différents laboratoires, ou encore différents pathologistes, etc.

De même, lors des opérations de rangement et de stockage des blocs ou des lames d'échantillons biologiques, notamment en vue d'éventuelles analyses comparatives ultérieures, des solutions d'archivage et de traçabilité doivent être mises en œuvre.

Des équipements destinés à la mise en œuvre de mesures de traçabilité dans le domaine des prélèvements biologiques sont d'ores et déjà disponibles sur le marché, et décrites notamment dans les publications FR 3 055 559 du présent demandeur ou EP 3 200 118.

L'objet de la présente invention est de proposer une solution alternative plus efficace, permettant d'améliorer la productivité de l'ensemble du personnel susceptible d'intervenir lors des phases de préparation, d'analyse et de rangement de prélèvements tissulaires ou cellulaires. Un autre but de l'invention est de proposer une solution permettant de faciliter la manipulation des supports d'échantillons biologiques, définis par les cassettes, les blocs de paraffine, et les lames, et d'en préserver l'intégrité structurelle tout au long du processus auquel ils sont soumis, en particulier lorsqu'il s'agit de lames.

A cet effet, la présente invention concerne un dispositif du genre indiqué en préambule, caractérisé en ce en ce qu'il comporte des moyens de reconnaissance d'un logement vide au sein dudit réceptacle définis par des moyens de détection d'une caractéristique physique du fond de l'alvéole ou d'un élément solidaire de ce fond.

Une telle caractéristique présente l'avantage de permettre un paramétrage des moyens de traitement informatique au terme duquel ces derniers délivrent à un opérateur en charge de l'archivage des échantillons biologiques, une information selon laquelle un logement donné est vide. L'opérateur peut alors déduire de cette information que l'absence de détection de données encodées dans ce même logement donné n'est pas due à une erreur de lecture de la part de l'appareil mais uniquement à une absence de support d'échantillon biologique. Il peut également être prévu de paramétrer les moyens de traitement informatique de manière telle qu'ils ne fournissent à l'opérateur aucune information quant à la vacuité d'un logement, et qu'une alerte n'est délivrée qu'en cas de constat, par les moyens de traitement informatique à la fois de la présence d'un support dans un logement et d'une absence de lecture des données encodées correspondantes.

En d'autres termes, l'objet de l'invention permet à l'opérateur de consacrer son temps à la correction d'éventuelles erreurs de lecture réellement commises par l'appareil de lecture, et par conséquent de lui éviter des tâches de vérification inutiles, observées lors de la mise en œuvre de certains équipements de l'art antérieur. En somme, grâce à une telle caractéristique, l'efficacité et par conséquent la productivité des opérateurs sont améliorées.

Conformément à une variante de réalisation envisageable du dispositif selon l'invention, il a été imaginé que le fond de l'alvéole peut par exemple comporter au moins un volume s'étendant en relief ou en creux sur sa face destinée à recevoir le support d'échantillon biologique, tandis que l'appareil est équipé de moyens de détection dudit volume, montés dans ladite cavité.

Une autre solution proposée dans le cadre de l'invention prévoit que l'appareil peut être équipé d'un capteur de niveau de gris monté dans ladite cavité, paramétré pour reconnaître un niveau de gris représentatif d'une alvéole vide.

Dans le même objectif, selon encore une autre alternative, le fond de chaque alvéole peut comporter des informations représentatives de sa vacuité, sous forme de données encodées.

Dans un but de préservation de l'intégrité structurelle d'une lame d'échantillon biologique, il a été prévu, dans un cas dans lequel chaque alvéole est conçue apte à loger en emboîtement un support d'échantillon biologique présentant une forme de lame parallélépipédique rectangle, que chaque alvéole présente de préférence une profondeur, comprise entre le bord supérieur de sa paroi périphérique et son fond, au moins égale à l'épaisseur d'une lame.

Par ailleurs, afin de faciliter la manipulation des échantillons biologiques, il a été prévu, que dans une telle variante de réalisation, le fond de l'alvéole présente un tronçon central plan prolongé par deux tronçons d'extrémité dont au moins un est incurvé de manière à ménager au moins une zone de jeu, s'étendant sous un plan passant par le tronçon central et délimitée par la paroi périphérique et le fond de l'alvéole.

D'autre part, conformément à une caractéristique préférentielle de l'invention, le fond est plan sur une longueur égale à 2/3 de la longueur d'une alvéole.

Le tronçon d'extrémité incurvé présente alors avantageusement un rayon de courbure tel qu'il permet un basculement d'une lame logée dans l'alvéole en appui de manière tangentielle le long du tronçon d'extrémité incurvé, de sorte à limiter au maximum le risque de brisure d'une lame lors de son extraction par un opérateur.

Conformément à une caractéristique additionnelle du dispositif selon l'invention, des moyens conçus aptes à garantir une insertion totale du réceptacle dans ladite cavité avant la mise en œuvre de l'appareil de lecture des données encodées et de détermination de données de localisation, peuvent être prévus.

Ces derniers peuvent par exemple être définis par une fourche optique dont l'appareil de lecture est équipé, et qui est conçue apte à détecter une paire d'orifices ménagés sur un bord du plateau.

Par ailleurs, il a également été imaginé que l'appareil de lecture peut être équipé de moyens d'identification du type de réceptacle inséré dans ladite cavité. L'avantage étant alors de conférer un caractère universel à l'appareil permettant de limiter le nombre d'équipements à acquérir par les laboratoires de recherche et d'analyse pour gérer leur stock d'échantillons biologiques.

La présente invention sera mieux comprise à la lecture de la description faite en référence aux figures annexées, fournies à titre d'exemples non limitatifs, et dans lesquelles :
- La figure 1 illustre une vue de dessus d'un plateau définissant une variante de réalisation du réceptacle selon l'invention, comportant une pluralité d'alvéoles pleines de lames d'échantillons biologiques et quelques alvéoles vides,

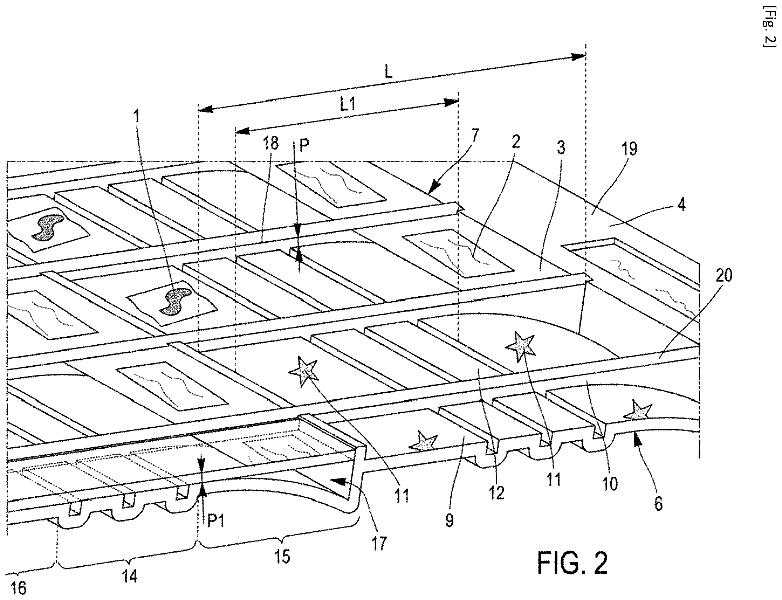

- La figure 2 représente une vue en coupe transversale du plateau de la figure 1,
- La figure 3 est une vue en coupe transversale du plateau de la figure 1 illustrant l'extraction d'une lame échantillonnée, et
- La figure 4 est une vue en coupe longitudinale de deux plateaux de la figure 1 superposés.

Comme indiqué ci-dessus, la présente invention a pour objet un dispositif pour le stockage et la traçabilité d'échantillons biologiques 1 prélevés sur un patient, un animal, voire une plante, en vue de travaux de recherche ou d'analyses médicales.

La description qui suit concerne l'application d'un tel dispositif à la traçabilité d'échantillons biologiques 1 munis d'informations d'identification sous forme de données encodées 2, au cours de leur phase d'analyse dans un service de pathologie, pendant laquelle ils sont véhiculés sur des lames 3, classiquement de forme parallélépipédique rectangle, stockées dans des réceptacles en forme de plateau 4 présentant une pluralité d'alvéoles 5 organisées en colonnes 6 et en rangées 7. Toutefois, il va de soi que le dispositif selon l'invention est étudié pour convenir pour l'ensemble des étapes de la vie de tels échantillons biologiques 1, depuis leur prélèvement jusqu'à leur destruction.

De manière classique, le dispositif selon l'invention comporte au moins un appareil (non illustré) comportant au moins une cavité conformée pour accueillir de manière temporaire un réceptacle, tel qu'un plateau 4, et équipé de moyens de lecture des données encodées 2 et de détermination de données relatives à la localisation de chaque lame 3 au sein du plateau 4. En l'occurrence, dans l'exemple illustré, ces dernières correspondent aux emplacements en termes de colonnes 6 et rangées 7 occupés par les alvéoles 5 dans lesquelles les lames 3 sont logées sur le plateau 4. Plus précisément, dans cet exemple, le plateau 4 contient quinze lames 3 d'échantillons biologiques 1, tandis que les alvéoles 5 occupant les rangées F, I, J de la colonne 1 et celles occupant les rangées E et G de la colonne 2 sont vides.

Par ailleurs, le dispositif selon l'invention comporte en outre des moyens de traitement informatiques (non illustrés) des données lues et déterminées par un tel appareil.

Conformément à l'invention, il comporte également des moyens de reconnaissance d'un logement vide au sein dudit réceptacle, et par conséquent d'une alvéole 5 vide au sein du plateau 4.

En référence aux figures, les alvéoles 5 sont conçues aptes à loger chaque lame 3 en emboîtement et sont délimitées par un fond 9 surmonté par une paroi périphérique 10. Afin de permettre la reconnaissance par l'appareil de lecture d'une alvéole 5 vide, différents moyens de détection d'une caractéristique physique du fond 9 d'une alvéole 5 ou d'un élément solidaire de ce fond 9 peuvent être prévus dans le cadre de l'invention.

Ainsi, selon une variante de réalisation envisageable, le fond 9 d'une alvéole 5 peut comporter deux volumes 11, par exemple en forme d'étoile, s'étendant en relief ou en creux sur sa face 12 destinée à recevoir la lame 3 supportant l'échantillon biologique 1, l'appareil de lecture étant alors équipé de moyens de détection dudit volume, montés dans ladite cavité de réception du plateau 3.

Conformément à une solution alternative, le fond 9 de chaque alvéole 5 peut comporter des informations représentatives de sa vacuité, par exemple sous forme de données encodées 13, imprimées sur des pastilles apposées sur le fond 9 ou imprimées directement sur le fond 9, et aptes à être lues par les moyens de lecture de données encodées que comporte l'appareil de lecture. Un détecteur de caractères de type « ROC » (reconnaissance optique de caractères) pourrait également être prévu, apte à conclure à une absence de lame 3, en cas de détection de caractères imprimés directement sur le fond 9 ou sur des étiquettes collées sur celui-ci (non illustrés).

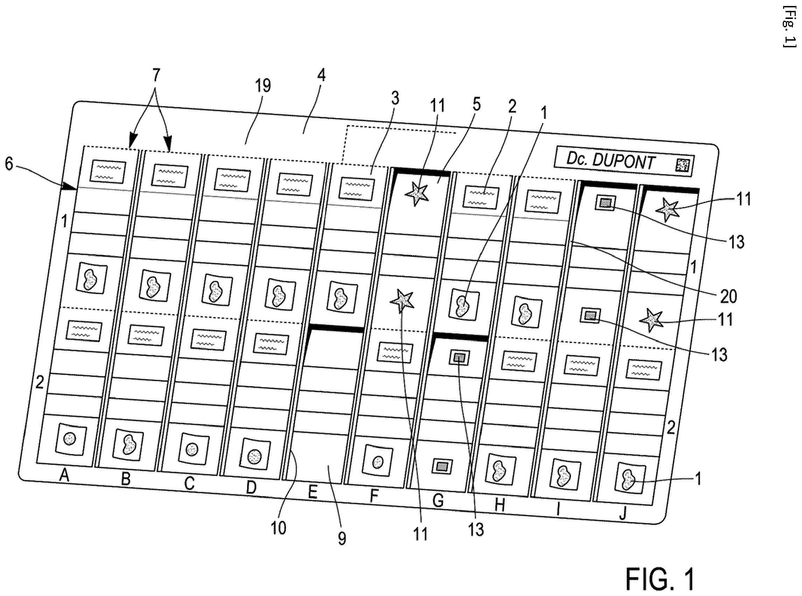

Il convient de noter que dans ces exemples, les volumes 11, données encodées 13, ou caractères imprimés, sont implantés sur le fond 9 des alvéoles 5 de manière à être masqués, d'une part par les données encodées 2 et d'autre part par l'échantillon biologique 1 que comportent les lames 3, lorsque celles-ci occupent les alvéoles 5 (cf. fig. 1). Ainsi, les volumes 11, données encodées 13, ou caractères imprimés ne sont détectables qu'en cas d'alvéole 5 vide et leur détection est par conséquent immédiatement interprétée par les moyens de traitement informatique comme représentative d'une telle alvéole 5 vide.

Une solution additionnelle proposée par la présente invention en vue de permettre la reconnaissance par l'appareil de lecture d'une alvéole 5 vide, consiste à équiper ce dernier d'un capteur de niveau de gris monté dans la cavité de réception du plateau 4, et paramétré pour reconnaître un niveau de gris représentatif d'une alvéole 5 vide, par définition différent du niveau de gris détecté en cas de présence d'une lame 3. En pratique, il sera fait appel, à cet effet, à la caméra dont l'appareil de lecture, que comporte classiquement le dispositif selon l'invention, est équipé. Ainsi, les moyens de traitement informatique de ce dernier seront paramétrés pour comparer les niveaux de gris d'une image d'un plateau 4 contenant des lames 3 relevée par la caméra, avec les niveaux de gris d'une image témoin relevée préalablement par cette même caméra alors que le plateau 4 était vide. Une correspondance entre les niveaux de gris d'une alvéole 5 de l'image témoin et les niveaux de gris d'une alvéole 5 d'un plateau 4 en cours de lecture étant synonyme d'absence de lame dans cette même alvéole, aucune information d'erreur de lecture ne sera délivrée, ce qui permet de dispenser l'opérateur d'une étape de vérification. En somme, une telle vérification ne s'avérera nécessaire que dans le cas où les moyens de traitement informatiques identifieront à la fois une erreur de lecture des données encodées 2 et une absence de correspondance entre les niveaux de gris d'une alvéole 5 de l'image témoin et les niveaux de gris d'une alvéole 5 d'un plateau 4 en cours de lecture, signifiant la présence d'une lame 3.

Dans le plateau 4 illustré aux figures, chaque alvéole 5 présente une profondeur p, comprise entre le bord supérieur 18 de sa paroi périphérique 18 et la face 12 du tronçon central 14 du fond 9, au moins égale à l'épaisseur p1 d'une lame 3 d'échantillon biologique 1. Ainsi, lorsqu'une telle lame 3 est logée dans une alvéole 5, elle ne présente aucune zone émergeant hors de cette dernière, susceptible d'être exposée à d'éventuels chocs ou raclements intempestifs risquant de la dégrader. Une telle caractéristique permet par conséquent d'assurer l'intégrité structurelle des lames 3 logées dans les alvéoles 5.

Afin d'améliorer la préhension d'une lame 3 logée dans une alvéole 5, le fond 9 de l'alvéole 5 a par ailleurs été conçu de manière à présenter un tronçon central plan 14 prolongé par deux tronçons d'extrémité 15, 16, dont le tronçon 16 est également plan, tandis que le tronçon 15 est incurvé de manière à ménager au moins une zone de jeu 17, s'étendant sous un plan passant par le tronçon central 14 et délimitée par la paroi périphérique 10 et le fond 9 de l'alvéole 5.

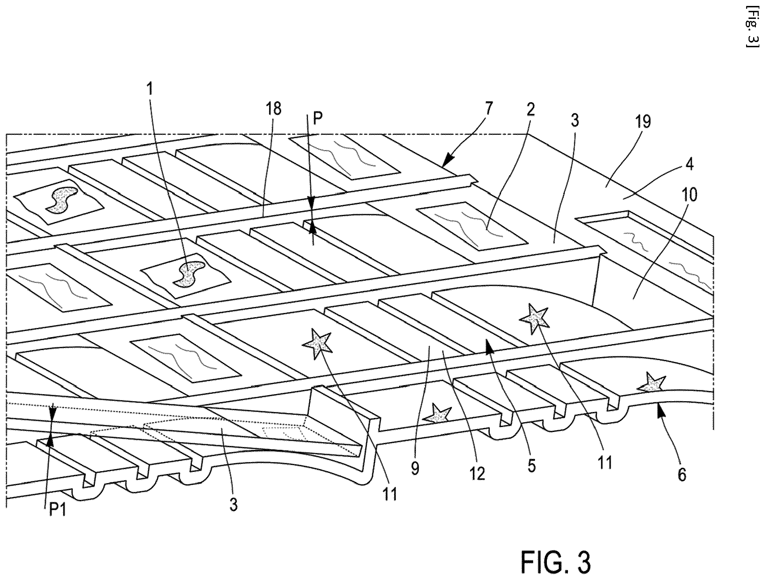

De plus, afin de garantir qu'au repos, une lame 3 logée dans une alvéole 5 demeure bien à plat, le tronçon d'extrémité 16 et le tronçon central 14 s'étendent ensemble sur une longueur L1 égale à 2/3 de la longueur L de l'alvéole 5. Tout risque de brisure d'une lame 3 lors de son extraction, lié à un déplacement trop brusque est évité grâce au fait que le tronçon d'extrémité 15 incurvé présente alors préférentiellement un rayon de courbure permettant un basculement d'une lame logée dans l'alvéole en appui de manière tangentielle le long du tronçon d'extrémité incurvé (cf. fig. 3). Bien entendu, une structure peut également être envisagée dans laquelle le fond de chaque alvéole n'est plan que le long de son tronçon central, tandis que ses deux tronçons d'extrémités sont incurvés. Dans ce cas, la préhension d'une lame est encore optimisée puisqu'elle peut s'opérer indifféremment par l'une ou l'autre de ses extrémités, avec le même niveau de délicatesse.

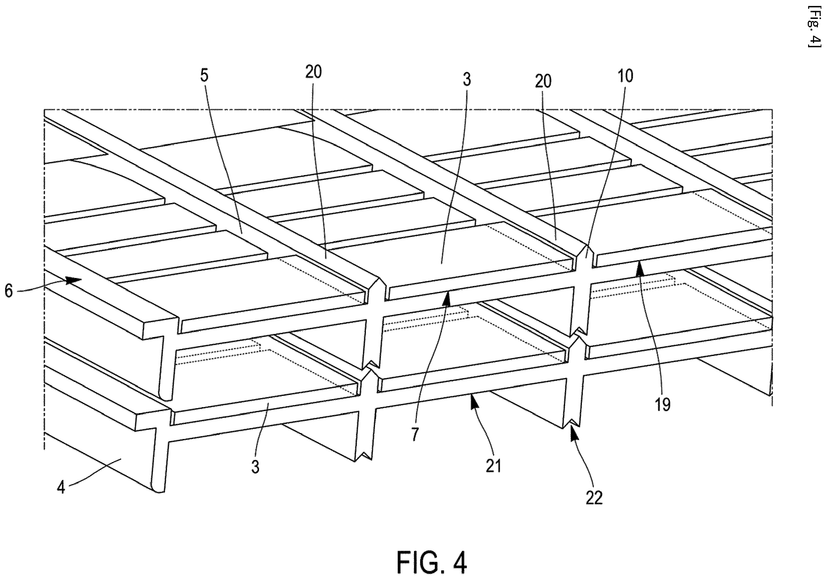

Tel qu'illustré à la figure 4, le plateau 4 est avantageusement conformé de manière à pouvoir être facilement empilé avec un plateau 4 identique. A cet effet, dans la variante de réalisation illustrée, sa face supérieure 19 comporte une pluralité de nervures 20 de section triangulaire, formées le long de chaque rangée 7 dans le prolongement de la paroi périphérique 10 de chaque alvéole 5, tandis que sa face inférieure 21 comporte une pluralité de gorges 22 de section en « V » complémentaire à celle des nervures 20, et s'étendant parallèlement à celles-ci, le long de pattes 23 prolongeant la face inférieure 21. Une telle caractéristique permet une meilleure organisation au sein des laboratoires d'analyse biologiques et une optimisation de l'espace disponible pour le rangement et le stockage des lames 3 tout en assurant leur protection.

Il convient encore de noter que le dispositif selon la présente invention est de préférence complété par des moyens conçus aptes à garantir une insertion totale d'un réceptacle tel que le plateau 4 dans la cavité qu'il comporte, avant la mise en œuvre de ses moyens de lecture des données encodées 2 13 et de détermination des données de localisation dont la nature a été précisée ci-dessus.

A cet effet, l'appareil de lecture peut par exemple être équipé d'une fourche optique conçue apte à détecter une paire d'orifices (non illustrés) ménagés sur un bord du plateau 4.

Par ailleurs, l'appareil de lecture peut être équipé de moyens d'identification du type de réceptacle inséré dans ladite cavité, de sorte à provoquer la mise en service, le cas échéant, de moyens de lecture dédiés à un type donné de réceptacle.

## Revendications

1. Dispositif pour le stockage et la traçabilité d'échantillons biologiques (1) placés dans des supports (3) munis d'informations d'identification sous forme de données encodées (2), ledit dispositif comportant :
- au moins un réceptacle (4) pourvu de logements (5) de stockage desdits supports (3), le réceptacle étant défini par un plateau (4) présentant une pluralité d'alvéoles (5) formant lesdits logements, configurées pour héberger chacune un support (3) d'échantillon biologique (1), délimitées par un fond (9) surmonté par une paroi périphérique (10),
- au moins un appareil de lecture des données encodées et de détermination de données relatives à la localisation de chaque support (3) au sein du réceptacle (4), comportant au moins une cavité conformée pour accueillir le réceptacle (4) de manière temporaire,
- des moyens de traitement informatiques des données lues et déterminées par ledit appareil,
et étant **caractérisé en ce qu'**il comporte des moyens de reconnaissance d'un logement (5) vide au sein dudit réceptacle (4) définis par des moyens de détection d'une caractéristique physique du fond (9) de l'alvéole (5) ou d'un élément solidaire de ce fond (9).

2. Dispositif selon la revendication 1, **caractérisé en ce que** le fond (9) de l'alvéole (5) comporte au moins un volume (11) s'étendant en relief ou en creux sur sa face (12) destinée à recevoir le support (3) d'échantillon biologique (1) tandis que l'appareil est équipé de moyens de détection dudit volume (11), montés dans ladite cavité.

3. Dispositif selon la revendication 1, **caractérisé en ce que** l'appareil est équipé d'un capteur de niveau de gris monté dans ladite cavité, paramétré pour reconnaître un niveau de gris représentatif d'une alvéole (5) vide.

4. Dispositif selon la revendication 1, **caractérisé en ce que** le fond (9) de chaque alvéole (5) comporte des informations représentatives de sa vacuité, sous forme de données encodées (13).

5. Dispositif selon l'une quelconque des revendications 1 à 4, **caractérisé en ce que** chaque alvéole (5) est conçue apte à loger en emboîtement un support (3) d'échantillon biologique (1) présentant une forme de lame (3) parallélépipédique rectangle, et **en ce qu'**elle présente une profondeur p, comprise entre le bord supérieur (18) de sa paroi périphérique (10) et son fond (9), au moins égale à l'épaisseur p1 d'une lame (3) d'échantillon biologique (1).

6. Dispositif selon la revendication 5, **caractérisé en ce que** le fond (9) de l'alvéole (5) présente un tronçon central plan (14) prolongé par deux tronçons d'extrémité (15, 16) dont au moins un est incurvé de manière à ménager au moins une zone de jeu (17), s'étendant sous un plan passant par le tronçon central (14) et délimitée par la paroi périphérique (10) et le fond (5) de l'alvéole (5).

7. Dispositif selon la revendication 6, **caractérisé en ce que** chaque alvéole (5) présente une longueur L tandis que le fond (9) est plan sur une longueur L1 égale à 2/3 de la longueur L de l'alvéole (5).

8. Dispositif selon l'une quelconque des revendications précédentes, **caractérisé en ce qu'**il comporte des moyens conçus aptes à garantir une insertion totale du réceptacle (4) dans ladite cavité avant la mise en œuvre de l'appareil de lecture des données encodées (2,13) et de détermination de données de localisation.

9. Dispositif selon la revendication 8, **caractérisé en ce que** l'appareil de lecture est équipé d'une fourche optique conçue apte à détecter une paire d'orifices ménagés sur un bord du plateau (4).

10. Dispositif selon l'une quelconque des revendications précédentes, **caractérisé en ce que** l'appareil de lecture est équipé de moyens d'identification du type de réceptacle inséré dans ladite cavité.
